# EUROPEAN PATENT APPLICATION

(11) **EP 1 862 118 A1**
(43) Date of publication of application: **05.12.2007**
(21) Application number: 05743905.1
(22) Date of filing: 27.05.2005
(51) Int. Cl.: A61B 6/00

(54) **RADIOGRAPHIC IMAGING SYSTEM**

(30) Priority: 25.03.2005 JP 2005088881
(71) Applicant: Konica Minolta Medical & Graphic, Inc., Tokyo 191-8511 (JP)
(72) Inventor: OHARA, Hiromu, 1928505 (JP)
(74) Representative: Henkel, Feiler & Hänzel
(86) International application number: PCT/JP2005/009743
(87) International publication number: WO 2006/103790

(57) **Abstract**

A radiographic imaging system having: a radiation image detector to obtain an image information; and a console to control the radiation image detector, wherein the radiation image detector includes a detector communication unit communicating to the console, and a detector control unit to which a signal from the detector communication unit is inputted, the console includes a console communication unit communicating to the detector communication unit, a console control unit controlling the console communication unit, and a reporting unit controlled by the console control unit, and after the console control unit controls the console communication unit to transmit a replying instruction signal for instructing a reply to the detector communication unit, the console control unit detects whether there is a replying signal based on the instructing, and when the replying signal is not inputted, the console control unit controls the reporting unit to report that a communication abnormality occurs.

## Description

### Technical Field

The present invention relates to a radiographic imaging system. In particular, the present invention relates to a radiographic imaging system for generating a radiation image as represented by an X-ray image.

### Background Art

So far, in a medical diagnosis, a radiation image which is obtained by irradiating a radiation such as an X-ray or the like to a subject and by detecting an intensity distribution of the radiation transmitted through the subject has been widely in use. These days, a radiographic imaging apparatus using an FPD (Flat Panel Detector; radiation image detector) that detects a radiation and converts the detected radiation into an electric energy to be detected as a radiation image information is being proposed upon radiography. In such a radiographic imaging apparatus, a diagnosis function to check a state of an image generating unit is provided for judging whether the image generation has been performed normally or not (for example, see JP-Tokukai-2004-73489A).

Further, these days, a cassette-type FPD in which the FPD is contained in a cassette for the purpose of improving a transportability and a handling ability of the FPD is being developed (for example, see Patent Document 1). In particular, in order to utilize the transportability of the FPD, a cassette-type FPD which is wirelessly communicates with a console for a control thereof is being proposed.
Patent Document 1: Japanese Patent Application Laid-Open Publication No. Hei6-342099

### Disclosure of the invention

### Problem to be Solved by the Invention

The above-mentioned diagnosis function is to check an operation of the image generation unit only. In this diagnosis mode, under a specific X-ray exposure condition, in order to judge whether an image generation is performed at certain desired dose over the whole part of a light receiving unit of the X-ray image generating apparatus or not, checking of a pixel output value is performed for the evaluation to see whether a pixel output value within a requirement has been obtained. However, by the conventional diagnosis function, it is not possible to check a communication state of the cassette-type FPD. In other words, even when the image generation unit normally functions, if a communication state of the cassette-type FPD is not normal, it is not possible to transmit a correct image. Therefore, the image generation needs to be re-performed. Fewer times of re-performance of an image generation are more desirable, in order not to give a patient unnecessary exposure to a patient.
In particular, since the cassette-type FPD is transportable, it is used at various locations such as an image generation room, a ward, an operating room and the like, without staying in a specific place to be used for an image generation. In other words, it is necessary to check the communication state at each location.

An object of the present invention is to make it possible to check a communication state of a radiation image detector at various locations and to prevent an unnecessary re-performance of an image generation.

### Means for Solving Problems

According to the invention of claim 1, a radiographic imaging system comprises:
a radiation image detector to obtain an image information; and
a console to control the radiation image detector,
wherein the radiation image detector comprises a detector communication unit to communicate to the console, and a detector control unit to which a signal from the detector communication unit is inputted,
the console comprises a console communication unit to communicate to the detector communication unit, a console control unit to control the console communication unit, and a reporting unit which is controlled by the console control unit, and
after the console control unit controls the console communication unit to transmit a replying instruction signal for instructing a reply to the detector communication unit, the console control unit detects whether there is a replying signal based on the instructing, and when the replying signal is not inputted to the console communication unit, the console control unit controls the reporting unit to report that a communication abnormality occurs.

According to the invention of claim 2, in the radiographic imaging system of claim 1, the console communication unit and the detector communication unit may be wirelessly communicated.

According to the invention of claim 3, in the radiographic imaging system of claim 1 or 2, after the console control unit controls the console communication unit to transmit the replying instruction signal to the detector communication unit, when the replying signal is not inputted to the console communication unit for not less than a predetermined period, the console control unit may judge that there is the communication abnormality.

According to the invention of claim 4, in the radiographic imaging system of any one of claims 1-3, when the radiation image detector is related to the console, the console control unit may control the console communication unit to transmit the replying instruction signal to the detector communication unit.

According to the invention of claim 5, in the radiographic imaging system of any one of claims 1-3, the console control unit may control the console communication unit to transmit the replying instruction signal to the detector communication unit when the console control unit transmits an image generation instruction signal to instruct an image generation to the radiation image detector.

According to the invention of claim 6, in the radiographic imaging system of any one of claims 1-5, the replying instruction signal may be a check signal for confirming an operation of each driving unit of the radiation image detector.

According to the invention of claim 7, in the radiographic imaging system of claim 6, the console control unit may control the console communication unit to transmit the check signal to the detector communication unit, when the console control unit transmits the image generation instruction signal to instruct the image generation to the radiation image detector.

### Effect of the Invention

Normally, when a radiation image detector is used, the detector is operated by a console installed in each of an image generation room, a hospital ward, an operating room and the like. In other words, although upon the use it is necessary to install the radiation image detector within a range within which the console is capable of communicating, when there is an abnormality in a communication state under such a condition, an image information obtained by the image generation is not transmitted to the console. Therefore, as the present invention, after the console control unit transmits a replying instruction signal for instructing a reply to the detector communication unit, the console control unit detects whether there is a replying signal based on the instruction. Then, when the replying signal is not inputted to the console communication unit, the console control unit controls the reporting unit to report that a communication abnormality occurs. Thereby, it is possible to recognize an abnormality of the communication state before an unnecessary image generation is performed. Consequently it is possible to reduce exposure dose to a patient by unnecessary re-performance of the image generation.

### Brief Description of the Drawings

FIG. 1 is a view showing a schematic structure exemplifying one embodiment of a radiographic imaging system relating to the present invention,
FIG. 2 is a perspective view showing a substantial structure of a radiation image detector relating to the present invention,
FIG. 3 is a block diagram showing the substantial structure of the radiation image detector relating to the present invention,
FIG. 4 is a view showing an equivalent circuit of a photoelectric conversion unit for processing one pixel, structuring a signal detecting unit provided in the radiation image detector of FIG. 2,
FIG. 5 is a view showing an equivalent circuit in which photoelectric conversion units of FIG. 4 are arranged two-dimensionally, and
FIG. 6 is a block diagram showing a substantial structure of a console structuring the radiographic imaging system of FIG. 1.

### Best Mode to Carry out the Invention

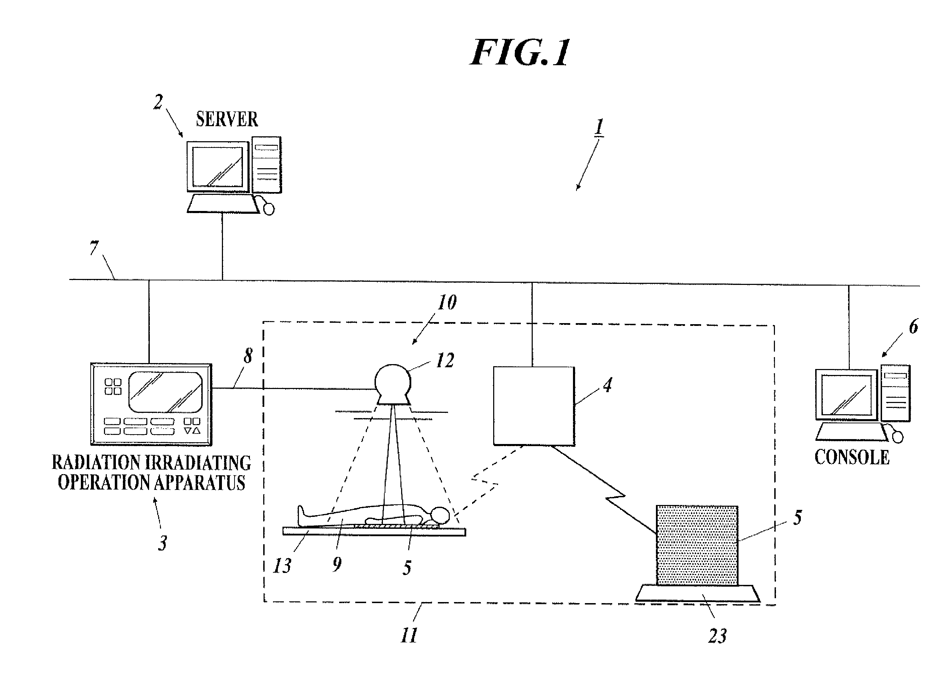

Hereinafter, an embodiment of the present invention will be described with reference to FIG. 1 to FIG. 6. FIG. 1 is a view showing a schematic structure of one embodiment of a radiographic imaging system to which a radiation image detector relating to the present invention is applied.

A radiographic imaging system 1 of the present embodiment is, for example, a system which is applied to a radiation image generation performed in a hospital. As shown in FIG. 1, the radiographic imaging system 1 comprises a server 2 for managing various information regarding an image generation, a patient and the like, a radiation irradiating operation apparatus 3 for performing an operation regarding a radiation image generation, a base station 4 for a communication according to a wireless communication system such as a wireless LAN (Local Area Network) or the like, and a console 6 for controlling a radiation image detector 5 and for performing an image processing or the like to a radiation image detected by the radiation image detector 5, wherein the server 2, the radiation irradiating operation apparatus 3, the base station 4 and the console 6 are connected to each other through a network 7. A radiation irradiating apparatus 10 for generating a radiation image by irradiating a radiation to a patient being a subject 9 is connected to the radiation irradiating operation apparatus 3 through a cable 8. For example, one pair of the radiation irradiating apparatus 10 and the radiation image detector 5 is installed in one image generation room. The radiation irradiating apparatus 10 is operated by the radiation irradiating operation apparatus 3 and the radiation image is detected by the radiation image detector 5, whereby it is possible to obtain radiation image information.

Here, the network 7 may be a communication line dedicated to the system. However, it is preferable to use an existing line such as Ethernet (trademark) or the like, because the dedicated communication line may decrease a flexibility of the system structure, or the like. Incidentally, in addition to the components mentioned above, a plurality of radiation irradiating operation apparatuses 3 for operating radiation irradiating apparatuses 10 in other image generation rooms 11, a plurality of radiation image detectors 5 and a plurality of consoles 6 may be connected to the network 7.

First, the radiation irradiating operation apparatus 3 comprises an input operation unit (not shown) comprising an operation panel and the like for operating the radiation irradiating apparatus 10 for inputting an image generation condition, for example, a tube voltage, a dose (mAs value) and the like, a display unit (not shown) for displaying an information such as the image generation condition and the like, and various instructions, etc, a power source (not shown) for supplying an electrical power to the radiation irradiating apparatus 10, and the like.

The radiation irradiating apparatus 10 is installed at the inside of the image generation room 11, and comprises a radiation source 12 for irradiating a radiation. Here, an X-ray is preferably used as the radiation. The radiation is irradiated from the radiation source 12 at a tube voltage and a dose set by the radiation irradiating operation apparatus 3. As the radiation source 12, for example, a radiation tube is used. The radiation tube accelerates an electron generated according to a thermal excitation by a high voltage, to be crashed to a cathode, whereby a radiation is generated.

Next, the radiation image detector 5 detects a radiation irradiated from the radiation source 12 of the radiation irradiating apparatus 10 and transmitted through the subject 9, for detecting a radiation image. The radiation image detector 5 is located within an irradiation area of the radiation that is irradiated from the radiation source upon the image generation. Here, as shown in FIG. 1, the radiation image detector 5 is, for example, located between the subject 9 and a platform 13 on which the subject is to be put. However, the location of the radiation image detector 5 is not limited to such a location. For example, it may be such that a detector attaching portion (not shown) to which the radiation image detector 5 is to be attached is provided below the platform, and the radiation image detector 5 is attached to the detector attaching portion.

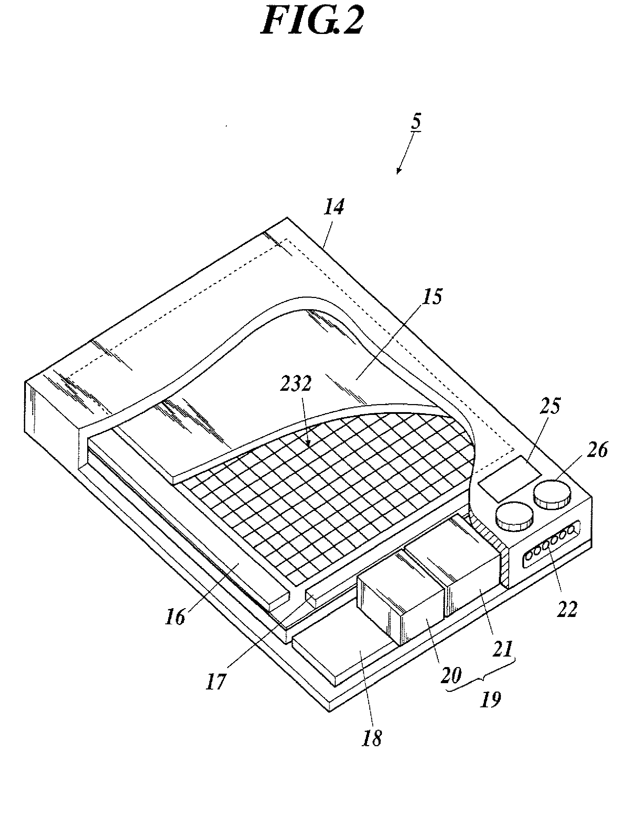

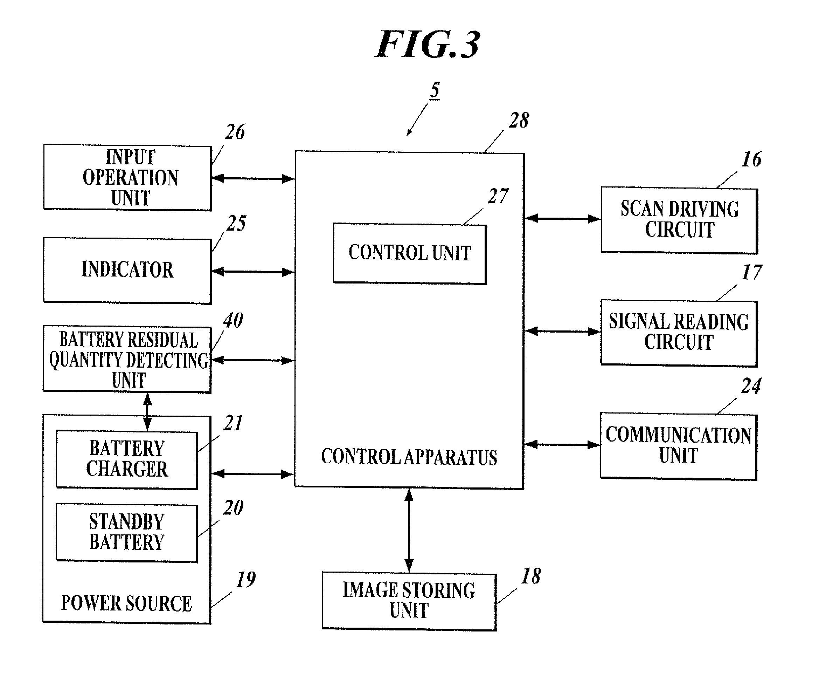

The radiation image detector 5 is a radiation image detector 5 being a cassette-type flat panel detector. Hereinafter, with reference to FIG. 2 and FIG. 3, a structure of the radiation image detector 5 will be described.

As shown in FIG. 2, the radiation image detector 5 comprises a chassis 14 for protecting the inside thereof. The radiation image detector 5 is structured to be portable as a cassette.
At the inside of the chassis 14, an imaging panel 15 for converting the irradiated radiation into an electric signal is so formed as to structure a layer. At a side of the imaging panel 15 where the radiation is irradiated, a luminescent layer (not shown) for emitting a light according to an intensity of the entered irradiation is provided.

The luminescent layer is in general called a scintillator layer. For example, the luminescent layer has a phosphor as a main component, and based on the entered radiation, the luminescent layer outputs an electromagnetic wave (light) having a wavelength from 300nm to 800nm, that is, an electromagnetic wave from an ultraviolet light through a visible light to an infrared light.
As a phosphor used in the luminescent layer, for example, a material having a parent body of CaWO₄ or the like, or a material having a parent body of Csl;Tl, Gd₂O2ₛ:Tb, TnS:Ag or the like in which a luminescence center material is activated can be used. Further, with a rare earth element defined as M, a phosphor represented by a general formula (Gd, M, Eu)₂O₂ can be used. In particular, it is preferable to use Csl:Tl or Gd₂O₂S:Tb since Csl:Tl and Gd₂O₂S:Tb have a high radiation absorbability and a high luminescence efficiency. By using Csl:Tl or Gd₂O₂S:Tb, it is possible to obtain a high quality image without too much noise.
On a surface being an opposite side to the surface to which the radiation of the luminescent layer is irradiated, formed is a signal detecting unit 232 for converting the electromagnetic wave (light) outputted from the luminescent layer into an electric energy to be accumulated and for outputting an image signal based on the accumulated electric energy.

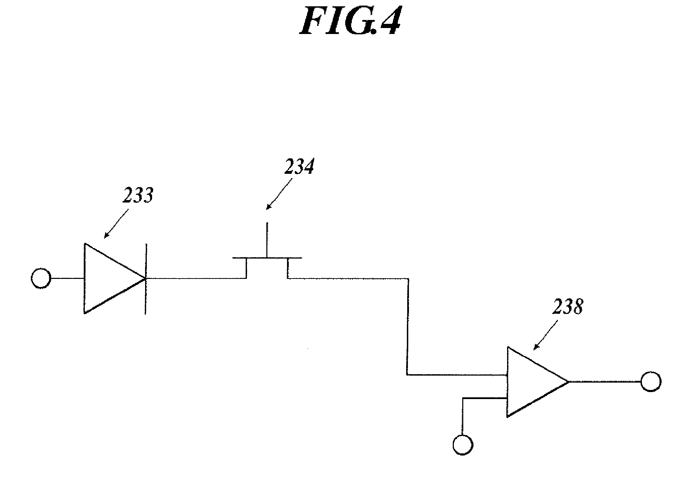

Here, a circuit structure of the imaging panel 15 will be described. FIG. 4 is a view showing an equivalent circuit of the photoelectric conversion unit for processing one pixel, structuring the signal detecting unit 232.
As shown in FIG. 4, the photoelectric conversion unit for processing one pixel comprises a photodiode 233, and a thin film transistor (hereafter, TFT 234) for picking up the electric energy accumulated in the photodiode 233 according to a switching. The picked-up electric signal is amplified by an amplifier 238 to a level at which a signal reading circuit 237 is capable of detecting. Here, the TFT 234 and a reset circuit (not shown) structured from a condenser are connected to the amplifier 238, and a reset operation for resetting the accumulated electric signal is performed at the reset circuit by switching the TFT 234 on. Further, the photodiode 233 may simply be a light condenser comprising a parasitic capacitance, or may be one including a juxtaposition of additional condensers so as to improve a dynamic range of the photodiode 233 and the photoelectric conversion unit. Here, the photodiode 233 may be one using an inorganic semiconductor system or one using an organic semiconductor system.

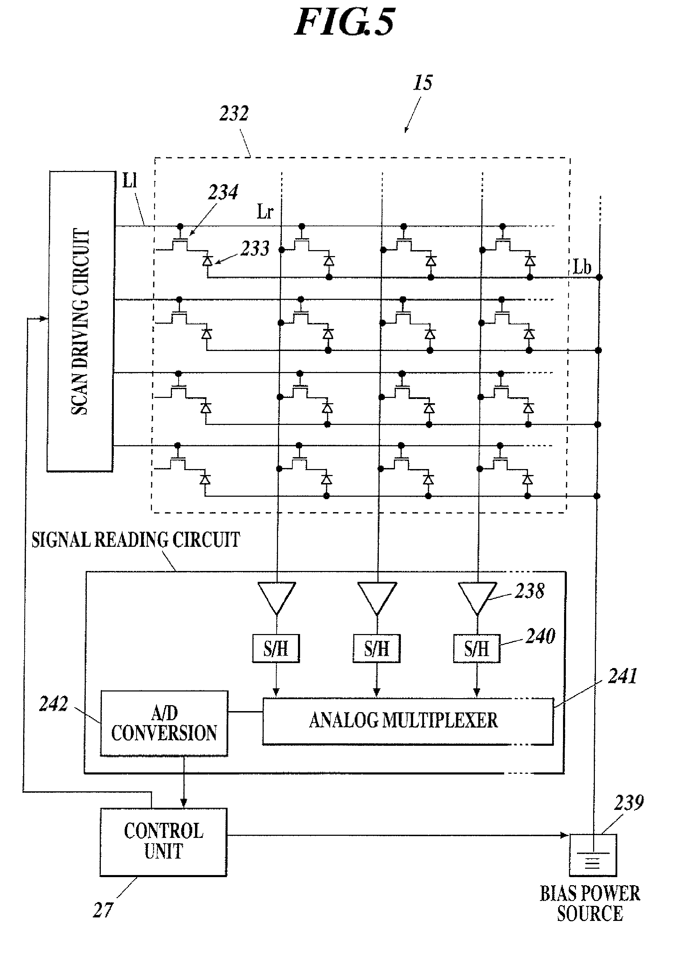

FIG. 5 is a vie showing an equivalent circuit in which such photoelectrical conversion units are arranged two-dimensionally, and a scanning line L1 and a signal line Lr are so arranged to be perpendicular to each other between the pixels. The TFT 234 is connected to the mentioned photodiode 233, and an edge of the photodiode 233 to which the TFT 234 is connected is connected to the signal line Lr. Meanwhile, another edge of the photodiode 233 is connected to edges of photodiodes 233 being adjacent to each other, and is connected to a bias power source 239 through a common bias line Lb. An edge of the bias power source 239 is connected to a control unit 27, and a voltage is applied to the photodiode 233 through the bias line Lb according to an instruction from the control unit 27. Further, the TFTs 234 arranged on each row are connected a common scan line L1, and the scan line L1 is connected to the control unit 27 through a scanning driving circuit 236. Similarly, the photodiodes 233 arranged on each column are connected to the common signal line Lr to be connected to the signal reading circuit 237 controlled by the control unit 27. In the signal reading circuit 237, provided on the common signal line Lr are the amplifier 238, a sample-and-hold circuit 240, an analog multiplexer 241 and an A/D converter 242, in the order from the one closest to the imaging panel 15.
Here, the TFT 234 may be one using an inorganic semiconductor system such as the one used in a liquid crystal display or the like, or one using an organic semiconductor system.
Further, in the present embodiment, a case in which the photodiode 233 as the photoelectrical conversion device is used is described. However, a CCD other than the photodiode may be used as the photoelectrical conversion device.

At the side of the signal detecting unit 232, provided are a scan driving circuit 16 for scanning and driving each photoelectrical conversion device by transmitting a pulse to each photoelectrical conversion device, and a signal reading circuit 17 for reading out an electric energy accumulated in each photoelectrical conversion device.
Further, as shown in FIG. 2 and FIG. 3, the radiation image detector 5 comprises an image storing unit 18 comprising a rewritable memory such as a nonvolatile memory (RAM), a flash memory or the like. The image storing unit 18 stores the image signal outputted from the imaging panel 15. The image storing unit 18 may be an incorporated-type memory, or a detachable memory such as a memory card.

Further, in the radiation image detector 5, a power source 19 is provided as an electric power supplying source for supplying an electric power to a plurality of driving units (the scan driving circuit 16, the signal reading circuit 17, a communication unit 24 (described later), the image storing unit (storing unit) 18, a control apparatus 28, a battery residual quantity detecting unit 40 (described later), an indicator 25 (described later), an input operation unit 26 (described later), the imaging panel 15 and the like) structuring the radiation image detector 5. The power source 19 comprises a standby battery 20 such as a manganese battery, an alkaline battery, an alkaline button battery, a lithium battery, a silver oxide battery, a zinc air battery, a nickel-cadmium battery, a mercury battery, a lead-acid battery or the like, and a chargeable battery charger (battery) 21 such as a nickel cadmium battery, a nickel hydrogen battery, a lithium ion battery, a small-type sealed lead acid battery, a lead accumulator, a fuel battery, a solar battery or the like. In this way, by comprising the standby battery 20 in addition to the battery charger 21, it is possible to supply at least a minimum electric power to the radiation image detector 5 even when the battery charger 21 is short of charged amount or even while the battery charger 21 is being changed. Therefore, it is possible to avoid erasing image information stored in the image storing unit 18 by mistake, or to avoid a state in which it is not possible to receive a signal from an external apparatus such as the console 6 or the like.

At an edge of the chassis 14, a terminal 22 for a charging purpose is formed. For example, as shown in FIG. 1, by attaching the radiation image detector 5 to a charging device 23 such as a cradle or the like, a terminal of the charging device 23 side and the terminal 22 of the chassis side are connected, and charging of the battery charger 21 is performed. Further, the battery charger 21 is exchangeable by taking it from a side part of the chassis 14. In addition, forms of the standby battery 20 and the battery charger 21 structuring the power source 19 are not limited to what is shown in FIG. 2. For example, a battery in a plate shape may be provided in parallel with the imaging panel 15. When each battery is provided in such a form, a ratio of the imaging panel surface with respect to the chassis 14 increases, and thereby it is possible to increase an effective imaging area. Therefore, it is possible to reduce a whole size of the radiation image detector 5 while the imaging area remains the same. As a result, it is possible to miniaturize the radiation image detector 5.

Further, in the radiation image detector 5, the communication unit 24 (detector communication unit: see FIG. 3) for transmitting/receiving various signals to/from an external apparatus such as the console 6 and the like, is provided. The communication unit 24, for example, transmits the image signal outputted from the imaging panel 15 to the console 6 and receives an image generation instruction signal, a standby instruction signal and the like transmitted from the console 6.

Further, at one edge of the surface of the chassis 14, the indicator 25 for displaying a charging status of the battery charger 21, various operation statuses and the like to be reported is provided. Thereby, it is possible for an operator to visually confirm the charging status of the battery charger 21 or the like of the radiation image detector 5.
At the outside of the chassis 14, the input operation unit 26 for inputting an image generation instruction and a standby instruction is provided.

Further, as shown in FIG. 3, the radiation image detector 5 comprises the control apparatus 28 comprising the control unit (detector control unit) 27 comprising a CPU, a ROM, a RAM and the like (none of them is shown).
The control unit 27 reads out a predetermined program stored in the RAM and develops the read program into an operation area of the RAM. Then, the CPU executes various processes according to the program. Thereby, the control unit 27 controls the plurality of driving units provided in the radiation image detector 5.
In the ROM of the control unit 27, various control data is stored in addition to the programs. The control data comprises, for example, residual quantity judging data for judging whether a residual quantity of the battery charger 21 satisfies a quantity with which an image generation is possible.

Further, the radiation image detector 5 comprises the battery residual quantity detecting unit 40 for detecting a residual quantity of the battery charger 21. The battery residual quantity detecting unit 40 detects a residual quantity of the battery charger 21 based on a control of the control unit 27, and outputs the obtained battery residual quantity to the control unit 27. It is possible to consider various timings for detecting the battery residual quantity. In the present embodiment at least, when an instruction to switch from a standby state to an image generation capable state (image generation instruction) is inputted from the input operation unit 26 or the communication unit 24, the control unit 27 controls the battery residual quantity detecting unit 40 to detect a residual quantity of the battery charger 21. Here, the standby state is a state in which an electric power consumption is less than that of the image generation capable state.

Then, the control unit 27 switches between the image generation capable state and the standby state based on a residual quantity detection result upon the input of the image generation instruction from the input operation unit 26 or the communication unit 24.
Then, information inputted from the input operation unit 26 and a signal received from the communication unit 24 is transmitted to the control unit 27, and the control unit 27 controls each unit based on the transmitted information and signal.
Further, the control unit 27 drives the scan driving circuit 16 to transmit a pulse to each photoelectric conversion device, for making each photoelectric conversion device scan and for driving each photoelectric conversion device. Then, the signal reading circuit 17 for reading out an electric energy accumulated in each photoelectric conversion device reads out an image signal, and the read image signal is transmitted to the control unit 27. The control unit 27 stores the transmitted image signal in the image storing unit 18. Further, the image signal stored in the image storing unit 18 is transmitted to the console 6 through the communication unit 24 accordingly.

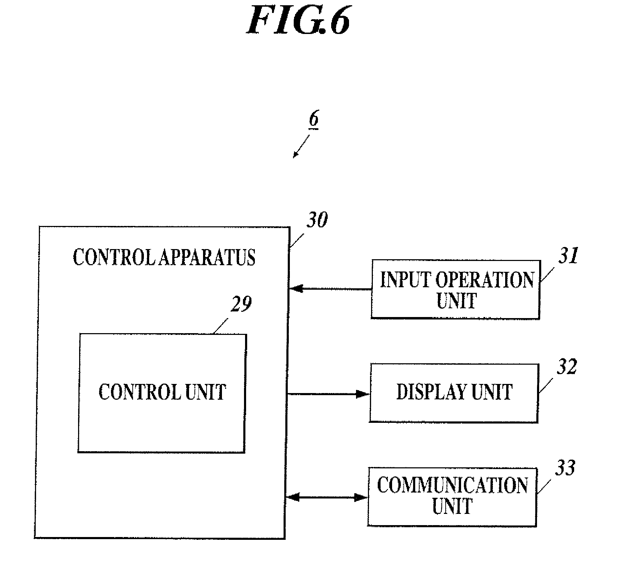

Next, as shown in FIG. 6, the console 6 comprises, for example, a control apparatus 30 comprising a control unit (console control unit) 29 comprising a generic CPU, a generic ROM, a generic RAM and the like (none of them is shown). The control unit 29 reads out a predetermined program stored in the ROM and develops the read program into an operation area of the RAM, and the CPU executes various processes according to the program.
Further, the console 6 comprises an input operation unit 31 for inputting various instructions and the like, a display unit (reporting unit) 32 for displaying an image, various messages and the like, a communication unit (console communication unit) 33 for transmitting/receiving a signal to/from an external device such as the radiation image detector 5 and the like.

The input operation unit 31 comprises, for example, an operation panel, a keyboard, a mouse and the like. The input operation unit 31 outputs a pushed signal of a key pushed on the operation panel or the keyboard by an operation to the control unit 29 as an input signal.

The display unit 32 comprises a CRT (Cathode Ray Tube), an LCD (Liquid Crystal Display) or the like, and displays various screens according to an instruction of a display signal outputted from the control unit 29.
The communication unit 33 is to communicate various information with the radiation image detector 5 through the base station 4 according to a wireless communication system such as a wireless LAN or the like.

To the control unit 29, a signal inputted from the input operation unit 31, a signal received from outside through the communication unit 33 and the like are transmitted, and the control unit 29 performs a predetermined process corresponding to the transmitted signal. For example, radiation image information detected by the radiation image detector 5 is transmitted to the control unit 29, and the control unit 29 performs a predetermined image process based on the transmitted radiation image information for obtaining a radiation image. Further, the control unit 29 displays on the display unit 32, a radiation image, a thumbnail image, various information inputted from the input operation unit 31, a signal indicating that an abnormality of the radiation image detector 5 occurs, a residual quantity of the battery charger 21 based on a detection result by the battery residual quantity detecting unit 40, a state of the radiation image detector 5 (image generation capable state or standby state) and the like.

Here, since the radiation image detector 5 is a portable type, the radiation image detector 5 is used in each room such as an image generation room, a hospital word, an operating room and the like, after the radiation image detector 5 is moved to such a room. The radiation image detector 5 is related to a specific console 6 before the movement. Then, upon being moved to another room, after the previous relation is released, the radiation image detector is related to a new console 6. At this time, since identification information of the radiation image detector 5 (for example, ID, IC tag, barcode and the like) is inputted from the input operation unit 31 of the console 6, the relation is made based on the identification information.

Further, although there is a case in which a plurality of radiation image detectors 5 are related to one console 6, since one radiation image detector 5 is used in a normal image generation, one image radiation image detector 5 is selected before the image generation and the selection is inputted from the input operation unit 31 to the control unit 29. The control unit 29 transmits an image generation instruction signal for instructing an image generation to a radiation image detector 5 corresponding to the selection result, among the plurality of radiation image detectors 5.

The control unit 29 of the console 6 controls the communication unit 33 to transmit a check signal to the communication unit 24 of the radiation image detector 5 when the relation with the radiation image detector 5 is done or when the image generation instruction signal is transmitted. The check signal is both a signal for instructing to confirm an operation of each driving unit (for example, a residual quantity detection of the battery charger 21, an operation of the image storing unit 18 and the like), and a replying instruction signal for instructing to reply to the communication unit 24 of the radiation image detector 5.

The control unit 29 detects whether there is a replying signal based on the replying instruction from the communication unit 24 of the radiation image detector 5. If the replying signal has not been inputted to the communication unit 33 for not less than a predetermined period, it is judged as a communication abnormality, and the control unit 29 controls the display unit 32 to report that a communication abnormality occurs.

Next, an operation of the radiographic imaging system 1 to which the radiation image detector 5 relating to the present embodiment will be described.

First, when the radiation image detector 5 is moved to a new location (image generation room or the like), a radiography technician inputs identification information of the radiation image detector 5 from the input operation unit 31 of the console 6. The control unit 29 of the console 6 relates the radiation image detector 5 based on the identification information. When the relation is made, the control unit 29 controls the communication unit 33 to transmit the check signal to the communication unit 24 of the radiation image detector 5. Based on the check signal, the control unit 27 of the radiation image detector 5 executes an operation confirmation of each driving unit. Here, since not receiving the check signal from the control unit 27 or not replying the replying signal means that a communication abnormality occurs in some way, the replying signal is not inputted to the communication unit 33 of the console 6 for not less than a predetermined period. If the replying signal has not been inputted for not less than the predetermined period, the control unit 29 of the console 6 judges that there is a communication abnormality, and the control unit 29 controls the display unit 32 to report that a communication abnormality occurs. The radiography technician deals with the communication abnormality based on the report.

When there is not communication abnormality or when a communication abnormality is resolved, an image generation is reserved. Normally, when an image generation reservation is not inputted to the radiation image detector 5, in order to be able to perform the image generation soon after the reservation is done, the control unit 27 of the radiation image detector 5 controls each operation state of the plurality of driving units to be in a standby state.

Thereafter, when the image generation reservation instruction is inputted to the console 6, the radiography technician selects one of the radiation image detector 5 to be used for the image generation on the console 6, and inputs accordingly to the input operation unit 31. The inputted content is transmitted to the communication unit 24 of the selected radiation image detector 5 through the communication unit 33 of the console 6, and inputted to the control unit 27, as image generation instruction information for example.

At this time, the control unit 29 of the console 6 controls the communication unit 33 to transmit the check signal to the communication unit 24 of the radiation image detector 5. The control unit 29 detects whether there is a replying signal from the communication unit 24 of the radiation image detector 5 based on the replying signal, and if a replying signal has not been inputted to the communication unit 33 of the console 6 for not less than a predetermined period, the control unit 29 judges that there is a communication abnormality, and then the control unit 29 controls the display unit 32 to report that a communication abnormality occurs. The radiography technician deals with the communication abnormality based on the report.

When a communication state is normal, the control unit 27 of the radiation image detector 5 controls an electrical power consumption quantity of the battery charger based on the image generation instruction information, for switching the standby state to the image generation capable state. Before the switching, the control unit 27 controls the battery residual quantity detecting unit 40 to detect a residual quantity of the battery charger 21. In addition, also when the radiography technician directly operates the input operation unit 26 of the radiation image detector 5 to input an image generation instruction, the control unit 27 controls each operation state of the plurality of driving units based on the image generation instruction to control the electric power consumption quantity of the battery charger 21, for switching the standby state to the image generation capable state.

As above, in accordance with the present embodiment, after the control unit 29 of the console 6 transmits the check signal to the communication unit 24 of the radiation image detector 5, the control unit 29 detects whether there is a replying signal based on the instruction. Then, if the replying signal has not been inputted to the communication unit 33 of the console 6, the control unit 29 controls the display unit 32 to report that a communication abnormality occurs. Therefore, it is possible to recognize an abnormality of a communication state before an unnecessary image generation is performed. Thereby, it is possible to avoid an unnecessary performance of an image generation and therefore to reduce an exposure dose to a patient. Further, since the radiation image detector 5 is used in various image generation rooms, a corresponding console 6 is changed at each time of changing an image generation room, and therefore it is necessary to check to see if a communication is normally done at each time. However, in the present embodiment, check of a communication is performed when the relation of the console 6 is switched, and as a result, it is possible to prevent a wrong image generation.

Further, a case in which a replying signal has not been inputted to the communication unit 33 of the console 6 for not less than a predetermined period after the check signal is transmitted to the communication unit 24 of the radiation image detector 5, it is judged that there is a communication abnormality. Therefore, it is possible to judge that there is a communication abnormality without waiting for an input of the replying signal for a long period.

When the relation with the radiation image detector 5 is done, the control unit 29 of the console 6 controls the communication unit 33 of the console 6 to transmit the check signal to the communication unit 24 of the radiation image detector 5. Therefore, it is possible to do the check of a communication state soon after the relation is done.

Then, when the control unit 29 of the console 6 transmits the image generation instruction signal to the radiation image detector 5, the control unit 29 controls the communication unit 33 of the console 6 to transmit the check signal to the communication unit 24 of the radiation image detector 5. Therefore, it is possible to do the check of a communication state before the image generation is performed.
A timing for the check is not limited to the timing in the present embodiment. It is also possible to do the check at a certain timing (according to a signal from the console 6 or the like) or to do the check every certain period.

In addition, needless to say, the present invention is not limited to the above-mentioned embodiment and may accordingly be changed.
For example, in the present embodiment, described is the case that a charging device such as a cradle or the like is used for charging the battery charger 21. However, the charging may be done by getting an electric power supply from an external power source by connecting a code for the electric power supply to a terminal of the radiation image detector. Further, it is also possible to have a structure in which the charging is done in a state where the battery charger is taken out from the radiation image detector.
Further, in the present embodiment, description was made by exemplifying the display unit for doing a visual reporting as a reporting unit relating to the present invention. However, a reporting unit may be one doing an auditory reporting.

### Industrial Applicability

As above, the radiographic imaging system of the present invention is useful for radiation image generation for which a cassette type radiation image detector is used, and especially suitable for performing radiation image generation by a radiation image detector which is moved to various locations.

### Description of Marks

- 1: radiographic imaging system
- 2: server
- 3: radiation irradiating operation apparatus
- 4: base station
- 5: radiation image detector
- 6: console
- 7: network
- 10: radiation irradiating apparatus
- 16: scan driving circuit
- 17: signal reading circuit
- 18: image storing unit
- 19: power source
- 21: chargeable battery charger
- 23: charging device
- 24: communication unit (detector communication unit)
- 25: indicator
- 26: input operation unit
- 27: control unit (detector control unit)
- 29: control unit (console control unit)
- 32: display unit (reporting unit)
- 33: communication unit (console communication unit)
- 40: battery residual quantity detecting unit

## Claims

1. A radiographic imaging system comprising:
a radiation image detector to obtain an image information; and
a console to control the radiation image detector,
wherein the radiation image detector comprises a detector communication unit to communicate to the console, and a detector control unit to which a signal from the detector communication unit is inputted,
the console comprises a console communication unit to communicate to the detector communication unit, a console control unit to control the console communication unit, and a reporting unit which is controlled by the console control unit, and
after the console control unit controls the console communication unit to transmit a replying instruction signal for instructing a reply to the detector communication unit, the console control unit detects whether there is a replying signal based on the instructing, and when the replying signal is not inputted to the console communication unit, the console control unit controls the reporting unit to report that a communication abnormality occurs.

2. The system of claim 1, wherein the console communication unit and the detector communication unit are wirelessly communicated.

3. The system of claim 1 or 2, wherein after the console control unit controls the console communication unit to transmit the replying instruction signal to the detector communication unit, when the replying signal is not inputted to the console communication unit for not less than a predetermined period, the console control unit judges that there is the communication abnormality.

4. The system of any one of claims 1-3, wherein when the radiation image detector is related to the console, the console control unit controls the console communication unit to transmit the replying instruction signal to the detector communication unit.

5. The system of any one of claims 1-3, wherein the console control unit controls the console communication unit to transmit the replying instruction signal to the detector communication unit when the console control unit transmits an image generation instruction signal to instruct an image generation to the radiation image detector.

6. The system of any one of claims 1-5, wherein the replying instruction signal is a check signal for confirming an operation of each driving unit of the radiation image detector.

7. The system of claim 6, wherein the console control unit controls the console communication unit to transmit the check signal to the detector communication unit, when the console control unit transmits the image generation instruction signal to instruct the image generation to the radiation image detector.
